# EUROPEAN PATENT SPECIFICATION

(11) **EP 4 482 443 B1**
(45) Date of publication and mention of the grant of the patent: **20.05.2026**
(21) Application number: 23706363.1
(22) Date of filing: 23.02.2023
(51) Int. Cl.: A61F 9/007, A61B 17/30, A61B 90/90, A61B 17/29

(54) **AN OPHTHALMIC SURGERY INSTRUMENT, AN ADD-ON MODULE, METHODS OF ASSEMBLING AND DE-ASSEMBLING AN OPHTHALMIC SURGERY INSTRUMENT**
AUGENCHIRURGISCHES INSTRUMENT, ZUSATZMODUL, VERFAHREN ZUR MONTAGE UND DEMONTAGE EINES AUGENCHIRURGISCHEN INSTRUMENTS
INSTRUMENT DE CHIRURGIE OPHTALMIQUE, MODULE COMPLÉMENTAIRE, PROCÉDÉS D'ASSEMBLAGE ET DE DÉSASSEMBLAGE D'UN INSTRUMENT DE CHIRURGIE OPHTALMIQUE

(30) Priority: 25.02.2022 NL 2031086
(43) Date of publication of application: 01.01.2025
(73) Proprietor: D.O.R.C. Dutch Ophthalmic Research Center (International) B.V., 3214 VN Zuidland (NL)
(72) Inventor: BERGWERF, Nadieh, 3214 VN Zuidland (NL); GÄHLER, Martinus Christianus, 3214 VN Zuidland (NL); KUNTZ, John Peter, 3214 VN Zuidland (NL); SCALI, Marta, 3214 VN Zuidland (NL); ROSENQUIST, Wim, 3214 VN Zuidland (NL)
(74) Representative: V.O.
(86) International application number: PCT/EP2023/054556
(87) International publication number: WO 2023/161342

(56) References cited:
- US-A- 5 893 873
- US-A1- 2017 100 114
- US-A1- 2021 059 702

## Description

The invention relates to an ophthalmic surgery instrument, comprising a handling unit having a longitudinal axis, a surgical module provided on the handling unit and extending away therefrom along the longitudinal axis thereof, and a shaft at least partially surrounding the surgical module, wherein the surgical module and the shaft are mutually movable relative along the longitudinal axis of the handling unit, a guiding element arranged at a distal end of the handling unit, the guiding element being provided with a central bore that is traversed by the surgical module and the shaft extending from the handling unit outwardly along the longitudinal axis thereof, wherein the handling unit comprises a driving unit for driving the surgical module or the shaft relative to the guiding element back and forth through the central bore of the guiding element, and an actuation unit associated with the driving unit for actuating the driving unit upon manual actuation of the actuation unit.

The above ophthalmic surgery instrument is known for example for ophthalmic surgery in the interior of the eye wherein the surgical module includes a movable distal portion such as a forceps. By providing a shaft partially surrounding the surgical module such that the shaft and the surgical module are movable relative to each other, the movable distal portion can selectively at least partly be covered or closed, or exposed or opened by the shaft, e.g. for actuating the surgical module and/or for inserting or removing the surgical module from the eye.

US 2021/059702 A1 discloses an ophthalmic surgery instrument, comprising a handling unit having a longitudinal axis, a surgical module provided on the handling unit and extending away therefrom along the longitudinal axis thereof, and a shaft at least partially surrounding the surgical module, wherein the surgical module and the shaft are mutually movable relative along the longitudinal axis of the handling unit.

There is an ongoing need to improve manual operation of the ophthalmic surgery instrument.

It is an object of the present invention to provide an ophthalmic surgery instrument having improved manual operation characteristics.

The invention is defined by claims 1, 9, 10, 11 and 12. Further embodiments of the invention are defined by the dependent claims.

Therefore, according to the invention, an ophthalmic surgery instrument is provided, comprising a handling unit having a longitudinal axis, a surgical module provided on the handling unit and extending away therefrom along the longitudinal axis thereof, and a shaft at least partially surrounding the surgical module, wherein the surgical module and the shaft are mutually movable relative along the longitudinal axis of the handling unit, a guiding element arranged at a distal end of the handling unit, the guiding element being provided with a central bore that is traversed by the surgical module and the shaft extending from the handling unit outwardly along the longitudinal axis thereof, wherein the handling unit comprises a driving unit for driving the surgical module or the shaft relative to the guiding element back and forth through the central bore of the guiding element, and an actuation unit associated with the driving unit for actuating the driving unit upon manual actuation of the actuation unit, wherein the actuation unit includes a distal slider at least partially surrounding the guiding element.

By providing the actuation unit with a distal slider at least partially surrounding the guiding element, operation of the instrument can be improved, e.g. wherein the actuation unit includes a plurality of actuation arms arranged around the longitudinal axis of the handling unit to improve stability and ease of use.

Moreover, the invention relates to an add-on module that can be assembled to an existing ophthalmic surgery device, thereby improving manual operation characteristics as well.

The invention also relates to a method of assembling and a method of de-assembling.

The invention further relates to an ophthalmic surgery instrument, wherein the handling unit comprises a proximal end portion rotatably mounted to a stationary portion, wherein one of the stationary portion and the proximal end portion is provided, along a circumferential direction around the longitudinal axis, with a multiple number of marks, while the other of the stationary portion and the proximal end portion is provided with an indication sign for selectively alignment, upon rotation of the proximal end portion relative to the stationary portion, with a mark of the multiple number of marks, thereby facilitating easy recognition the type of the surgical module 3 especially during preparation or use of the ophthalmic surgery instrument.

Further advantageous embodiments according to the invention are described in the following claims.

It should be noted that the technical features described above or below may each on its own be embodied in an ophthalmic surgery instrument and/or in a method, i.e. isolated from the context in which it is described, separate from other features, or in combination with only a number of the other features described in the context in which it is disclosed. Each of these features may further be combined with any other feature disclosed, in any combination.

The invention will be further elucidated on the basis of exemplary embodiments which are represented in the drawings. The exemplary embodiments are given by way of non-limitative illustrations of the invention. In the drawings:
Fig. 1 shows a schematic perspective view of an ophthalmic surgery instrument according to the invention;
Fig. 2 shows a schematic cross sectional view of ophthalmic surgery instrument shown in Fig. 1;
Fig. 3 shows a schematic cross sectional view of a distal end of the ophthalmic surgery instrument shown in Fig. 2, wherein actuation arms are in a decompressed state;
Fig. 4 shows a schematic cross sectional view of a distal end of the ophthalmic surgery instrument shown in Fig. 2, wherein actuation arms are in a compressed state;
Fig. 5 shows a schematic cross sectional view of a distal portion of another ophthalmic surgery instrument according to the invention;
Fig. 6 shows a flow chart of a method of assembling according to the invention;
Fig. 7 shows a flow chart of a method of de-assembling according to the invention, and
Fig. 8 shows a schematic perspective view of a further ophthalmic surgery instrument according to the invention.

In the figures identical or corresponding parts are represented with the same reference numerals. The drawings are only schematic representations of embodiments of the invention.

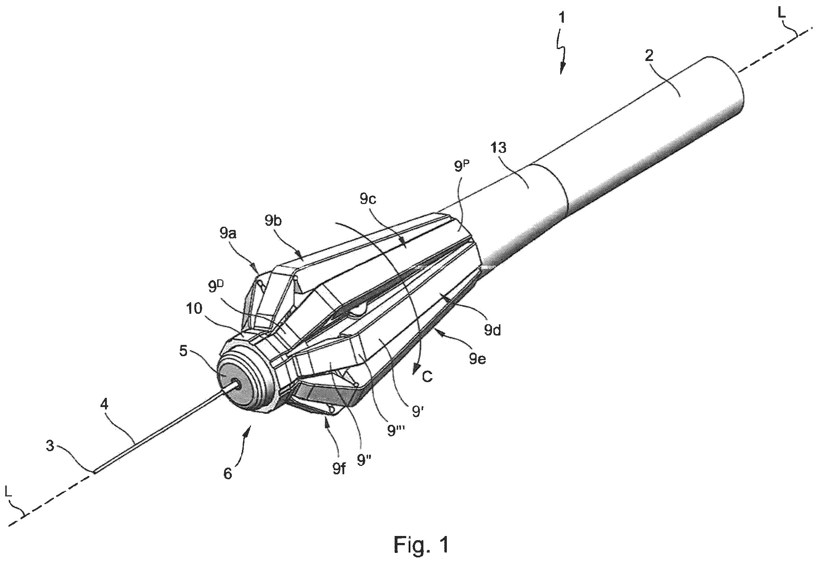

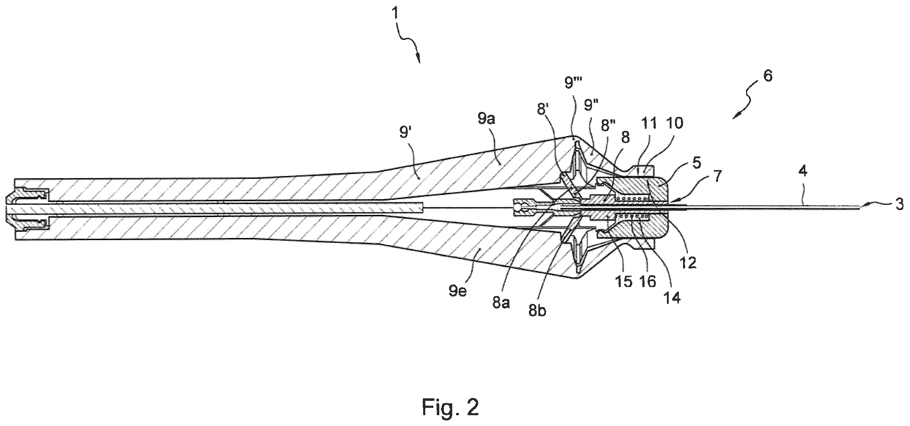

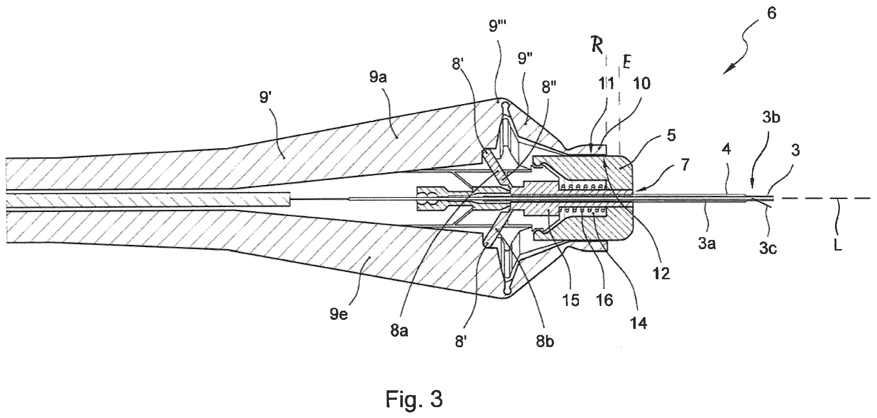

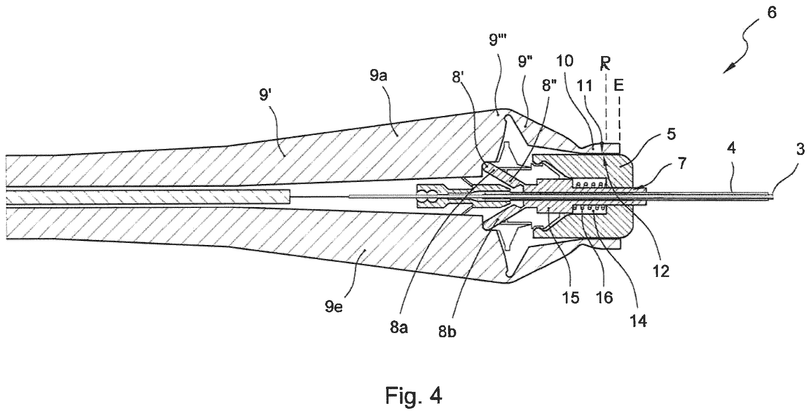

Figure 1 shows a schematic perspective view of an ophthalmic surgery instrument 1 according to the invention. Figure 2 shows a schematic cross sectional view thereof. The instrument 1 includes a handling unit 2 having a longitudinal axis L. Further, the instrument 1 includes a surgical module 3 provided on the handling unit 2 and extending away therefrom along the longitudinal axis L thereof, and a shaft 4 that at least partially surrounds the surgical module 3. The surgical module 3, see also Figs. 3 and 4, and the shaft 4 are mutually movable along the longitudinal axis L of the handling unit 2.

The ophthalmic surgery instrument 1 also includes a guiding element 5 arranged at a distal end 6 of the handling unit 2, the guiding element 5 being provided with a central bore 7 that is traversed by the surgical module 3 and the shaft 4 extending from the handling unit 2 outwardly along the longitudinal axis L thereof.

The handling unit 2 comprises a driving unit 8 for driving the surgical module 3 or the shaft 4 relative to the guiding element 5 back and forth through the central bore 7 of the guiding element 5. The handling unit 2 further comprises an actuation unit 9 associated with the driving unit 8 for actuating the driving unit 8 upon manual actuation of the actuation unit 9. The actuation unit 9 includes a distal slider 10 that at least partially surrounds the guiding element 5 in a circumferential direction C.

In the shown embodiment, the distal slider 10 is formed as a closed annular element completely surrounding the guiding element 5. In principle, the distal slider 10 may be non-closed in the circumferential direction, e.g. for assembling reasons. Preferably, a radial inner surface 11 of the distal slider 10 has a geometry matching a radial outer surface 12 of the guiding element 5, such as a circular or polygon cylinder surface.

Further, the actuation unit 9 is arranged for moving the distal slider 10 freely along the guiding element 5 along the longitudinal axis L upon manual actuation of the actuation unit 9. The distal slider may have a free end, at its distal side. In the shown embodiment, the guiding element 5 is stationary relative to the handling unit 2.

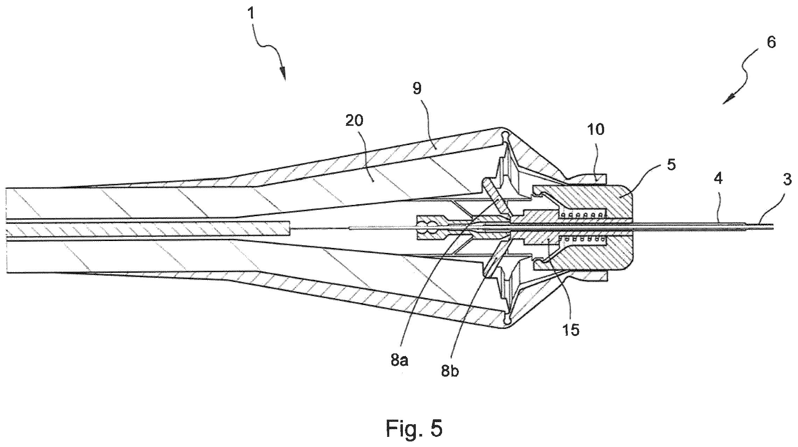

Further, in the embodiment shown in Figs. 1-4, the actuation unit 9 directly actuates the driving mechanism 8, i.e. upon actuation of the actuation unit 9, the actuation unit 9 contacts the driving mechanism 8 inducing a movement thereof. Alternatively, the actuation unit 9 indirectly actuates the driving mechanism 8, e.g. via an intermediate part as explained in more detail referring to Fig. 5.

As shown in Figs. 1 and 2, the actuation unit 9 includes a plurality of actuation arms 9a-e arranged along the circumferential direction C around the longitudinal axis L of the handling unit 2. The actuation arms 9, also referred to as basket, are adjustable between a compressed state and a decompressed state, wherein the actuation arms 9 in the compressed state are closer to the longitudinal axis L of the handling unit 2 than in the decompressed state.

Figure 3 shows a schematic cross sectional view of the distal end 6 of the ophthalmic surgery instrument shown in Fig. 2, wherein the actuation arms 9 are in a decompressed state;

Figure 4 shows a schematic cross sectional view of a distal end 6 of the ophthalmic surgery instrument shown in Fig. 2, wherein actuation arms 9 are in a compressed state.

In the decompressed state, as shown in Fig. 3, the distal slider 10 is located in a rest axial position R along the longitudinal axis L of the handling unit 2. On the other hand, in the compressed state, as shown in Fig. 4, the distal slider 10 is driven to an extended axial position E along the longitudinal axis L of the handling unit 2, away from the handling unit 2 offset relative to the rest axial position R. As described in more detail below, in the shown embodiment, in the compressed state, the shaft 4 is driven along the surgical module 3, away from the handling unit 2.

The guiding element 5 has a radial outer contour or radial outer surface 12, and the distal slider is arranged to slide along said radial outer contour or radial outer surface 12 along the longitudinal axis L upon manual actuation of the actuation unit 9.

The actuation arms 9 each include at least two arm segments 9', 9" and at least a hinge 9‴ interconnecting the at least two arm segments 9', 9", the arm segments 9', 9" having a stiffness that is larger than a stiffness of the hinge 9‴. By providing the actuation arms with portions having different stiffness, a bendable arm structure is realized. By pressing the arms radially inwardly, at least portions of the arms 9 bend radially inwardly. The arm segments 9', 9" can be made separately and assembled to each other, via the hinge 9"', or, alternatively, can be made as an integral part having locally different stiffness values. In principle, further arm segments can be added to the two segment arm structure, e.g. via an additional hinge. A local stiffness can be set by making the arm thinner or thicker and/or by changing its material. As a further alternative, the actuation arm can be formed without hinge 9"', however preferably such that the actuation arm bends upon manual actuation.

The actuation arms 9 can be designed in a way to provide different material properties to set the haptic conditions of the mechanism.

Further, the actuation arms 9 or basket can be personalized for instance as a 2k injected part with variable and different hardness and/or flexibility of the at least 2 arm segments 9', 9".

The actuation unit 9 further includes a proximal stationary element 13. Each of the actuation arms 9 has a proximal end 9^{P} and a distal end 9^{D}, the arm proximal ends 9^{P} being connected to the proximal stationary element 13, and the arm distal ends 9^{D} being connected to the distal slider 10, e.g. via respective intermediate hinges.

In the compressed state, as shown in Fig. 4, the distal slider 10 is driven to an extended axial position E along the longitudinal axis L of the handling unit 2, further remote from the proximal stationary element 13 than in the decompressed state, and further remote from the rest axial position R described referring to Fig 3.

As shown in Figs. 2-4, the driving unit 8 includes a radial to axial linkage, having a set of pivoting joint elements 8a-b, also referred to as wings, each pivoting joint element 8a-b having a first end 8' actuated by the actuation unit 9, and a second end 8" associated with the surgical module 3 or the shaft 4. The number of pivoting joint elements 8 may be the same as the number of actuation arms 9, such that each actuation arm 9 actuates a corresponding pivoting joint element 8. However, in principle, the number of pivoting joint elements 8 may be less than the number of actuation arms 9, e.g. two pivoting joint element 8.

Further, in the shown embodiment, the guiding element 5 includes a cavity 14 aligned with the central bore 7, wherein the driving unit 8 further includes an annular holding element 15 received in the cavity 14 of the guiding element 5, the annular holding element 15 being fixedly attached to the surgical module 3 or the shaft 4 and associated with the second ends 8" of the pivoting joint elements 8 or wings.

Upon adjusting the actuation arms 9 from the decompressed state towards the compressed state, by manually compressing the actuation arms 9, the second end 8" of the pivoting joint elements 8 moves along the longitudinal axis L of the handling unit 2, away from the proximal stationary element 13. In this process, the first ends 8' of the pivoting joint elements 8 pivot with respect to the second ends 8" towards the longitudinal axis L.

In the show embodiment, the actuation unit 9 and the driving unit 8 are arranged for driving the shaft 4, in particular for driving the shaft 4 away from the handling unit 2 upon manual actuation of the actuation unit 9. The surgical module 3 is stationary relative to the handling unit 2. In an alternative embodiment, the actuation unit 9 and the driving unit 8 are arranged for driving the surgical module 3. Then, the shaft may be stationary relative to the handling unit 2.

The cavity 14 further includes a spring element 16 for biasing the annular holding element 15 towards the handling unit 2. Similarly, the actuation arms 9 are biased towards the decompressed state. Then, the actuation arms 9 are in the decompressed state or are at least tending towards said decompressed state, when not manually actuated.

The surgical module 3 is formed as an integral part or as an assemblage of separate parts. As shown in Fig. 3, in an exemplary embodiment, the surgical module 3 has a rod 3a that is provided, at a distal end 3b thereof, with an operating unit 3c, such as a forceps, pair of scissors, fibre, scraper, vertical scissor or fiber assembly. At least one the actuation arms 9 of the actuation unit may have an outer or exterior contour deviating from an outer or exterior contour of other actuation arms, such that the at least one actuation arm is aligned with a circumferential orientation of the operating unit of the surgical module 3, e.g. the orientation of the forceps. By moving the shaft 4 relative to the surgical module 3, the distal end 3b can selectively be exposed or opened so as to actuate or operate the operating unit 3c, e.g. for grabbing and/or releasing tissue.

Figure 5 shows a schematic cross sectional view of a distal end 6 of another ophthalmic surgery instrument 1 according to the invention. Here, the instrument 1 includes an intermediate transfer unit 20 arranged between the actuation unit 9 and the driving unit 8. The actuation unit 9 may be fixed or removable from the intermediate transfer unit 20. Upon removing the actuation unit 9, the instrument 1 can be used by manually operating the intermediate transfer unit 20 directly.

Similarly, the actuation unit 9 can be added, as an add-on module, to an instrument 1 wherein the actuation 9 has been removed earlier.

Further, the actuation unit 9 can be added, as an add-on module to another type of instrument wherein the intermediate transfer unit 20 has originally been designed to be manually operated for driving the driving unit 8. The intermediate transfer unit may include a plurality of actuation arms arranged around the longitudinal axis L of the handling unit 2, such as two actuation arms only, instead of the basket type actuation unit 9 described above.

Generally, an add-on module for adding on an ophthalmic surgery device comprises an actuation unit to be associated with the intermediate transfer unit for actuating the intermediate transfer unit upon manual actuation of the actuation unit, wherein the actuation unit includes a distal slider at least partially surrounding the guiding element.

Here, the ophthalmic surgery device to which the add-on module may be added comprises:
- a handling unit having a longitudinal axis,
- a surgical module provided on the handling unit and extending away therefrom along the longitudinal axis thereof, and
- a shaft at least partially surrounding the surgical module,
   wherein the surgical module and the shaft are mutually movable relative along the longitudinal axis of the handling unit,

- a guiding element arranged at a distal end of the handling unit, the guiding element being provided with a central bore that is traversed by the surgical module and the shaft extending from the handling unit outwardly along the longitudinal axis thereof,
   wherein the handling unit comprises
- a driving unit for driving the surgical module or the shaft relative to the guiding element back and forth through the central bore of the guiding element, and
- an intermediate transfer unit associated with the driving unit for actuating the driving unit upon actuation of the intermediate transfer unit.

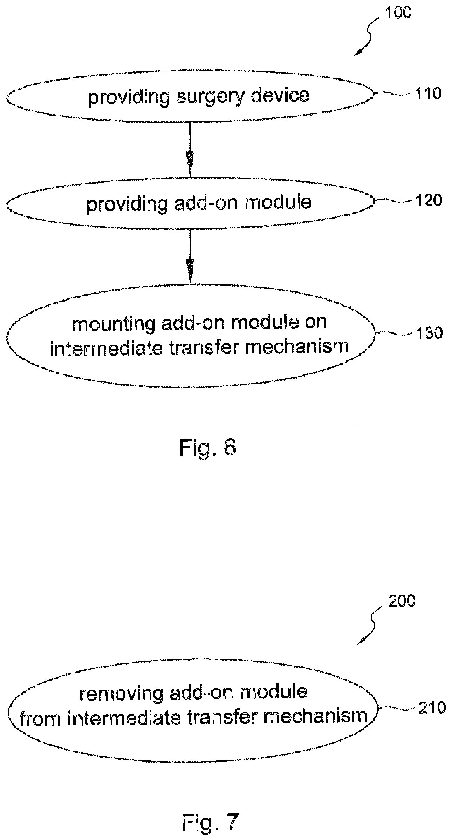

Figure 6 shows a flow chart of a method 100 according to the invention. The method is used for assembling an ophthalmic surgery instrument. The method 100 comprises a step of
- providing 110 an ophthalmic surgery device, comprising:
   - a handling unit having a longitudinal axis,
   - a surgical module provided on the handling unit and extending away therefrom along the longitudinal axis thereof, and
   - a shaft at least partially surrounding the surgical module, wherein the surgical module and the shaft are mutually movable relative along the longitudinal axis of the handling unit,
   - a guiding element arranged at a distal end of the handling unit, the guiding element being provided with a central bore that is traversed by the surgical module and the shaft extending from the handling unit outwardly along the longitudinal axis thereof, wherein the handling unit comprises
   - a driving unit for driving the surgical module or the shaft relative to the guiding element back and forth through the central bore of the guiding element, and
   - an intermediate transfer unit associated with the driving unit for actuating the driving unit upon actuation of the intermediate transfer unit,
- providing 120 an add-on module comprising an actuation unit to be associated with the intermediate transfer unit, wherein the actuation unit includes a distal slider at least partially surrounding the guiding element, and
- mounting 130 the add-on module on the intermediate transfer unit such that upon manual actuation of the actuation unit the intermediate transfer unit is actuated.

Figure 7 shows a flow chart of another method 200 according to the invention. The method 200 is used for de-assembling an ophthalmic surgery instrument. The method 200 comprises a step of removing the add-on module defined above from the intermediate transfer unit of the ophthalmic surgery device defined above.

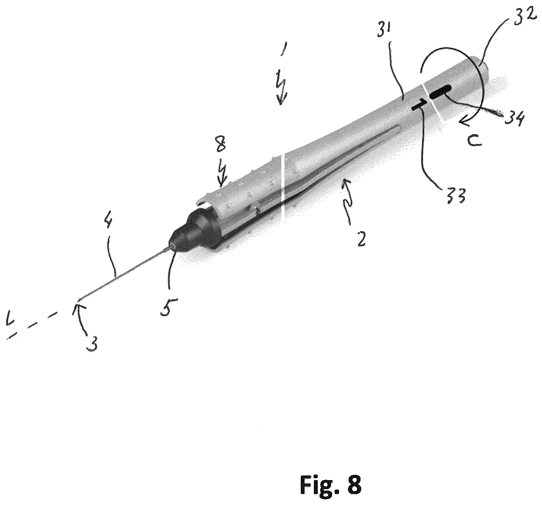

Figure 8 shows a schematic perspective view of a further ophthalmic surgery instrument 1 according to the invention. The instrument 1 has a handling unit 2 with a longitudinal axis L, a surgical module 3, a shaft 4 at least partially surrounding the surgical module 3 and a guiding element 5 as described referring to Fig. 1. The handling unit 2 comprises a driving unit 8 for driving the surgical module 3 or the shaft 4 relative to the guiding element 5 back and forth through the central bore of the guiding element 5 as described above.

The driving unit 8 further comprises a stationary portion 31 mainly extending around the longitudinal axis L, and a proximal end portion 32 rotatably mounted to the stationary portion 31, in particular in the circumferential direction C, around the longitudinal axis L. The stationary portion 31 may be mainly stationary in the circumferential direction C relative to the driving unit 8.

In the shown embodiment, the stationary portion 31 is provided, along the circumferential direction C around the longitudinal axis, with a multiple number of marks 33. Preferably, the marks 33 are substantially evenly distributed over the circumference of the stationary portion 31, along the circumferential direction C. The marks 33 may be visible, e.g. using an optical pattern or sign, e.g. "1", "2", and "3". Alternatively or additionally, the marks 33 may include a local deformation of the exterior surface of the stationary portion 31, e.g. a single or multiple number of inwardly or outwardly formed notches or bumps.

Further, the proximal end portion 32 is provided with an indication sign 34 for selectively alignment, upon rotation of the proximal end portion 32 relative to the stationary portion 31, with a mark 33 of the multiple number of marks 33. The multiple number of marks 33 corresponds to a type of the surgical module 3, respectively, such as a regular or specialty forceps type surgical module, or a regular or specialty scissor type surgical module. As an example, a first mark may correspond to a regular forceps type surgical module, a second mark may correspond to a regular scissor type surgical module, and a third mark may correspond to a specialty forceps or scissor type surgical module.

Similar to the marks 33, the indication sign 34 may be visible, optically distinguishable, e.g. using an optical pattern or sign and/or a local deformation of the exterior surface of the proximal end portion 32.

Upon rotation of the proximal end portion 32 relative to the stationary portion 31, a mark 33 corresponding to an actual type of the surgical module 3 may be selected. Then, the type of the surgical module 3 can be easily recognized by surgery personnel, during preparation or use of the ophthalmic surgery instrument 1.

As an alternative to embodiment shown in Fig. 8, the location of the multiple number of marks 33 and the indication sign 34, respectively, may be interchanged. Then, the multiple number of marks 33 are provided on the proximal end portion 32, while the indication sign 34 is provided on the stationary portion 31.

Preferably, the proximal end portion 32 is lockable in the circumferential direction C, i.e. the proximal end portion 32 can be locked to rotate in the circumferential direction C relative to the stationary portion 31, at least after selectively aligning the indication sign 34 to the selected mark 33, thereby counteracting that a further rotation causes the indication sign 34 to align with another mark not corresponding to the actual type of surgical module thereby reducing a chance that confusion or misunderstanding of the type of surgical module 3 may occur.

The invention is not restricted to the embodiments described herein. It will be understood that many variants are possible.

These and other embodiments will be apparent for the person skilled in the art and are considered to fall within the scope of the invention as defined in the following claims. For the purpose of clarity and a concise description features are described herein as part of the same or separate embodiments. However, it will be appreciated that the scope of the invention may include embodiments having combinations of all or some of the features described.

## Claims

1. An ophthalmic surgery instrument (1), comprising:
- a handling unit (2) having a longitudinal axis (L),
- a surgical module (3) provided on the handling unit (2) and extending away therefrom along the longitudinal axis (L) thereof, and
- a shaft (4) at least partially surrounding the surgical module (3),
wherein the surgical module (3) and the shaft (4) are mutually movable relative along the longitudinal axis (L) of the handling unit (2),
- a guiding element (5) arranged at a distal end (6) of the handling unit (2), the guiding element (5) being provided with a central bore (7) that is traversed by the surgical module (3) and the shaft extending from the handling unit outwardly along the longitudinal axis thereof,
wherein the handling unit (2) comprises
- a driving unit (8) for driving the surgical module (3) or the shaft (4) relative to the guiding element (5) back and forth through the central bore (7) of the guiding element (5), and
- an actuation unit (9) associated with the driving unit (8) for actuating the driving unit (8) upon manual actuation of the actuation unit (9),
wherein the actuation unit (9) includes a distal slider (10) at least partially surrounding the guiding element (5).

2. An ophthalmic surgery instrument according to claim 1, wherein the actuation unit is arranged for moving the distal slider freely along the guiding element along the longitudinal axis upon manual actuation of the actuation unit.

3. An ophthalmic surgery instrument according to any of the preceding claims, wherein the actuation unit directly actuates the driving mechanism.

4. An ophthalmic surgery instrument according to any of the preceding claims 1-3, further comprising an intermediate transfer unit coupled between the actuation unit and the driving unit.

5. An ophthalmic surgery instrument according to any of the preceding claims, wherein the guiding element has a radial outer contour and wherein the distal slider is arranged to slide along said radial outer contour along the longitudinal axis upon manual actuation of the actuation unit.

6. An ophthalmic surgery instrument according to any of the preceding claims, wherein the guiding element includes a cavity aligned with the central bore, and wherein the driving unit further includes a radial to axial linkage provided with a set of pivoting joint elements, each pivoting joint element having a first end actuated by the actuation unit and a second end associated with the surgical module or the shaft, the driving unit further including an annular holding element received in the cavity of the guiding element, the annular holding element being fixedly attached to the surgical module or the shaft and associated with the second ends of the pivoting joint elements.

7. An ophthalmic surgery instrument according to any of the preceding claims, wherein the surgical module has a rod that is provided, at a distal end thereof, with an operating unit, such as a forceps.

8. An ophthalmic surgery instrument according to claim 7, wherein the actuation unit includes a plurality of actuation arms arranged around the longitudinal axis of the handling unit, and wherein at least one the actuation arms of the actuation unit has an outer contour deviating from an outer contour of other actuation arms, the at least one actuation arm being aligned with a circumferential orientation of the operating unit of the surgical module.

9. An add-on module for adding on an ophthalmic surgery device (1), the device (1) comprising:
- a handling unit (2) having a longitudinal axis (L),
- a surgical module (3) provided on the handling unit (2) and extending away therefrom along the longitudinal axis (L) thereof, and
- a shaft (4) at least partially surrounding the surgical module (3), wherein the surgical module (3) and the shaft (4) are mutually movable relative along the longitudinal axis (L) of the handling unit (2),
- a guiding element (5) arranged at a distal end (6) of the handling unit (2), the guiding element (5) being provided with a central bore (7) that is traversed by the surgical module (3) and the shaft (4) extending from the handling unit outwardly along the longitudinal axis thereof,
wherein the handling unit (2) comprises
- a driving unit (8) for driving the surgical module (3) or the shaft (4) relative to the guiding element (5) back and forth through the central bore (7) of the guiding element (5), and
- an intermediate transfer unit (20) associated with the driving unit (8) for actuating the driving unit (8) upon actuation of the intermediate transfer unit (20),
wherein the add-on module comprises an actuation unit (9) to be associated with the intermediate transfer unit (20) for actuating the intermediate transfer unit (20) upon manual actuation of the actuation unit (9), and wherein the actuation unit (9) includes a distal slider (10) at least partially surrounding the guiding element (5).

10. A method of assembling an ophthalmic surgery instrument (1), comprising the steps of:
- providing an ophthalmic surgery device, comprising:
- a handling unit (2) having a longitudinal axis (L),
- a surgical module (3) provided on the handling unit (2) and extending away therefrom along the longitudinal axis (L) thereof, and
- a shaft (4) at least partially surrounding the surgical module (3), wherein the surgical module (3) and the shaft (4) are mutually movable relative along the longitudinal axis (L) of the handling unit (2),
- a guiding element (5) arranged at a distal end (6) of the handling unit (2), the guiding element (5) being provided with a central bore (7) that is traversed by the surgical module (3) and the shaft (4) extending from the handling unit outwardly along the longitudinal axis thereof,
wherein the handling unit (2) comprises
- a driving unit (8) for driving the surgical module (3) or the shaft (4) relative to the guiding element (5) back and forth through the central bore (7) of the guiding element (5), and
- an intermediate transfer unit (20) associated with the driving unit (8) for actuating the driving unit (8) upon actuation of the intermediate transfer unit (20),
- providing an add-on module comprising an actuation unit (9) to be associated with the intermediate transfer unit (20), wherein the actuation unit (9) includes a distal slider (10) at least partially surrounding the guiding element (5), and
- mounting the add-on module on the intermediate transfer unit (20) such that upon manual actuation of the actuation unit (9) the intermediate transfer unit (20) is actuated.

11. A method of de-assembling an ophthalmic surgery instrument, comprising a step of removing the add-on module defined in claim 9 from the intermediate transfer unit of the ophthalmic surgery device defined in claim 10.

12. An ophthalmic surgery instrument (1), comprising:
- a handling unit (2) having a longitudinal axis (L),
- a surgical module (3) provided on the handling unit (2) and extending away therefrom along the longitudinal axis (L) thereof, and
- a shaft (4) at least partially surrounding the surgical module (3), wherein the surgical module (3) and the shaft (4) are mutually movable relative along the longitudinal axis (L) of the handling unit (2),
- a guiding element (5) arranged at a distal end (6) of the handling unit (2), the guiding element (5) being provided with a central bore (7) that is traversed by the surgical module (3) and the shaft (4)extending from the handling unit outwardly along the longitudinal axis thereof,
wherein the handling unit (2) comprises
- a driving unit (8) for driving the surgical module (3) or the shaft (4) relative to the guiding element (5) back and forth through the central bore (7) of the guiding element (5),
- a stationary portion (31) mainly extending around the longitudinal axis (L), and
- a proximal end portion (32) rotatably mounted to the stationary portion (31), in particular around the longitudinal axis (L), wherein one of the stationary portion (31) and the proximal end portion (32) is provided, along a circumferential direction around the longitudinal axis (L), with a multiple number of marks (33), while the other of the stationary portion (31) and the proximal end portion (32) is provided with an indication sign (34) for selectively alignment, upon rotation of the proximal end portion (32) relative to the stationary portion (31), with a mark (33) of the multiple number of marks (33).

13. An ophthalmic surgery instrument according to claim 12, wherein the multiple number of marks corresponds to a type of the surgical module, respectively, in particular a regular or specialty forceps type surgical module, or a regular or specialty scissor type surgical module.

14. An ophthalmic surgery instrument according to claim 12 or 13, wherein the multiple number of marks are provided on the stationary portion.

15. An ophthalmic surgery instrument according to any of the preceding claims 12-14, wherein the proximal end portion is lockable in the circumferential direction, at least after the selectively alignment to the mark.

## Patentansprüche

1. Augenchirurgisches Instrument (1), umfassend:
- eine Handhabungseinheit (2) mit einer Längsachse (L),
- ein chirurgisches Modul (3), bereit gestellt an der Handhabungseinheit (2) und sich davon entlang der Längsachse (L) hiervon weg erstreckend, und
- einen Schaft (4), der das chirurgische Modul (3) zumindest teilweise umgibt, wobei das chirurgische Modul (3) und der Schaft (4) entlang der Längsachse (L) der Handhabungseinheit (2) relativ zueinander beweglich sind,
- ein Führungselement (5), angeordnet an einem distalen Ende (6) der Handhabungseinheit (2), wobei das Führungselement (5) mit einer zentralen Bohrung (7) versehen ist, die vom chirurgischen Modul (3) und dem Schaft durchquert wird, der sich von der Handhabungseinheit nach außen entlang deren Längsachse erstreckt,
wobei die Handhabungseinheit (2) umfasst:
- eine Antriebseinheit (8) zum Antreiben des chirurgischen Moduls (3) oder des Schafts (4) relativ zum Führungselement (5) hin und her durch die zentrale Bohrung (7) des Führungselements (5), und
- eine Betätigungseinheit (9), verbunden mit der Antriebseinheit (8), um die Antriebseinheit (8) bei manueller Betätigung der Betätigungseinheit (9) zu betätigen, wobei die Betätigungseinheit (9) einen distalen Schieber (10) einschließt, der das Führungselement (5) zumindest teilweise umgibt.

2. Augenchirurgisches Instrument nach Anspruch 1, wobei die Betätigungseinheit angeordnet ist, zum Bewegen des distalen Schiebers der Betätigungseinheit frei entlang des Führungselements entlang der Längsachse bei manueller Betätigung.

3. Augenchirurgisches Instrument nach einem der vorstehenden Ansprüche, wobei die Betätigungseinheit den Antriebsmechanismus direkt betätigt.

4. Augenchirurgisches Instrument nach einem der vorstehenden Ansprüche 1 bis 3, ferner umfassend eine Zwischenübertragungseinheit, gekoppelt zwischen der Betätigungseinheit und der Antriebseinheit.

5. Augenchirurgisches Instrument nach einem der vorstehenden Ansprüche, wobei das Führungselement eine radiale Außenkontur hat und wobei der distale Schieber angeordnet ist, um bei manueller Betätigung der Betätigungseinheit entlang der radialen Außenkontur entlang der Längsachse zu gleiten.

6. Augenchirurgisches Instrument nach einem der vorstehenden Ansprüche, wobei das Führungselement einen mit der zentralen Bohrung ausgerichteten Hohlraum einschließt und wobei die Antriebseinheit ferner eine radiale zu axiale Verbindung einschließt, versehen mit einem Satz von Schwenkgelenkelementen, wobei jedes Schwenkgelenkelement ein erstes Ende, betätigt durch die Betätigungseinheit, und ein zweites Ende, verbunden mit dem chirurgischen Modul oder dem Schaft, hat, wobei die Antriebseinheit ferner ein ringförmiges Halteelement einschließt, aufgenommen in den Hohlraum des Führungselements, wobei das ringförmige Halteelement starr mit dem chirurgischen Modul oder dem Schaft verbunden und mit den zweiten Enden der Schwenkgelenkelemente verbunden ist.

7. Augenchirurgisches Instrument nach einem der vorstehenden Ansprüche, wobei das chirurgische Modul einen Stab hat, der an einem distalen Ende davon mit einer Bedienungseinheit, wie einem Forceps, versehen ist.

8. Augenchirurgisches Instrument nach Anspruch 7, wobei die Betätigungseinheit eine Vielzahl von Betätigungsarmen einschließt, angeordnet um die Längsachse der Handhabungseinheit, und wobei mindestens einer der Betätigungsarme der Betätigungseinheit eine Außenkontur hat, die von einer Außenkontur anderer Betätigungsarme abweicht, wobei der mindestens eine Betätigungsarm mit einer Umfangsausrichtung der Bedieneinheit des chirurgischen Moduls ausgerichtet ist.

9. Zusatzmodul zum Anbringen an einer augenchirurgischen Vorrichtung (1), wobei die Vorrichtung (1) umfasst:
- eine Handhabungseinheit (2) mit einer Längsachse (L),
- ein chirurgisches Modul (3), bereit gestellt an der Handhabungseinheit (2) und sich davon entlang der Längsachse (L) hiervon weg erstreckend, und
- einen Schaft (4), der das chirurgische Modul (3) zumindest teilweise umgibt, wobei das chirurgische Modul (3) und der Schaft (4) entlang der Längsachse (L) der Handhabungseinheit (2) relativ zueinander beweglich sind,
- ein Führungselement (5), angeordnet an einem distalen Ende (6) der Handhabungseinheit (2), wobei das Führungselement (5) mit einer zentralen Bohrung (7) versehen ist, die vom chirurgischen Modul (3) und dem Schaft (4) durchquert wird, der sich von der Handhabungseinheit nach außen entlang deren Längsachse erstreckt,
wobei die Handhabungseinheit (2) umfasst:
- eine Antriebseinheit (8) zum Antreiben des chirurgischen Moduls (3) oder des Schafts (4) relativ zum Führungselement (5) hin und her durch die zentrale Bohrung (7) des Führungselements (5), und
eine Zwischenübertragungseinheit (20), verbunden mit der Antriebseinheit (8), zum Betätigen der Antriebseinheit (8) bei Betätigung der Zwischenübertragungseinheit (20),
- wobei das Zusatzmodul eine Betätigungseinheit (9) umfasst, die mit der Zwischenübertragungseinheit (20) zu verbinden ist, zum Betätigen der Zwischenübertragungseinheit (20) bei manueller Betätigung der Betätigungseinheit (9), und wobei die Betätigungseinheit (9) einen distalen Schieber (10) einschließt, der das Führungselement (5) zumindest teilweise einschließt.

10. Verfahren zum Montieren eines augenchirurgischen Instruments (1), umfassend die Schritte von:
- Bereitstellen einer augenchirurgischen Vorrichtung, umfassend:
- eine Handhabungseinheit (2) mit einer Längsachse (L),
- ein chirurgisches Modul (3), bereit gestellt an der Handhabungseinheit (2) und sich davon entlang der Längsachse (L) hiervon weg erstreckend, und
- einen Schaft (4), der das chirurgische Modul (3) zumindest teilweise umgibt, wobei das chirurgische Modul (3) und der Schaft (4) entlang der Längsachse (L) der Handhabungseinheit (2) relativ zueinander beweglich sind,
- ein Führungselement (5), angeordnet an einem distalen Ende (6) der Handhabungseinheit (2), wobei das Führungselement (5) mit einer zentralen Bohrung (7) versehen ist, die vom chirurgischen Modul (3) und dem Schaft durchquert wird, der sich von der Handhabungseinheit nach außen entlang deren Längsachse erstreckt,
wobei die Handhabungseinheit (2) umfasst:
- eine Antriebseinheit (8) zum Antreiben des chirurgischen Moduls (3) oder des Schafts (4) relativ zum Führungselement (5) hin und her durch die zentrale Bohrung (7) des Führungselements (5), und
- eine Zwischenübertragungseinheit (20), verbunden mit der Antriebseinheit (8), zum Betätigen der Antriebseinheit (8) bei Betätigung der Zwischenübertragungseinheit (20),
- Bereitstellen eines Zusatzmoduls, umfassend eine Betätigungseinheit (9), die mit der Zwischenübertragungseinheit (20) zu verbinden ist, wobei die Betätigungseinheit (9) einen distalen Schieber (10) einschließt, der das Führungselement (5) zumindest teilweise umgibt, und
- Montieren des Zusatzmoduls an der Zwischenübertragungseinheit (20), sodass bei manueller Betätigung der Betätigungseinheit (9) die Zwischenübertragungseinheit (20) betätigt wird.

11. Verfahren zum Demontieren eines augenchirurgischen Instruments, umfassend einen Schritt zum Entfernen des Zusatzmoduls definiert in Anspruch 9 von der Zwischenübertragungseinheit der augenchirurgischen Vorrichtung definiert in Anspruch 10.

12. Augenchirurgisches Instrument (1), umfassend:
- eine Handhabungseinheit (2) mit einer Längsachse (L),
- ein chirurgisches Modul (3), bereit gestellt an der Handhabungseinheit (2) und sich davon entlang der Längsachse (L) hiervon weg erstreckend, und
- einen Schaft (4), der das chirurgische Modul (3) zumindest teilweise umgibt, wobei das chirurgische Modul (3) und der Schaft (4) entlang der Längsachse (L) der Handhabungseinheit (2) relativ zueinander beweglich sind,
- ein Führungselement (5), das an einem distalen Ende (6) der Handhabungseinheit (2) angeordnet ist, wobei das Führungselement (5) mit einer zentralen Bohrung (7) versehen ist, die vom chirurgischen Modul (3) und dem Schaft (4) durchquert wird, der sich von der Handhabungseinheit nach außen entlang der Längsachse davon erstreckt,
wobei die Handhabungseinheit (2) umfasst:
- eine Antriebseinheit (8) zum Antreiben des chirurgischen Moduls (3) oder des Schafts (4) relativ zum Führungselement (5) hin und her durch die zentrale Bohrung (7) des Führungselements (5),
- einen feststehenden Abschnitt (31), der sich hauptsächlich um die Längsachse (L) erstreckt, und
- einen proximalen Endabschnitt (32), drehbar angebracht an dem feststehenden Abschnitt (31), insbesondere um die Längsachse (L), wobei einer von dem feststehenden Abschnitt (31) und dem proximalen Endabschnitt (32) entlang einer Umfangsrichtung um die Längsachse (L) mit einer Vielzahl von Markierungen (33) versehen ist, während der andere des feststehenden Abschnitts (31) und des proximalen Endabschnitts (32) mit einem Anzeigesymbol (34) versehen ist, um bei Drehung des proximalen Endabschnitts (32) relativ zum feststehenden Abschnitt (31) selektiv eine Markierung (33) der Vielzahl von Markierungen (33) auszurichten.

13. Augenchirurgisches Instrument nach Anspruch 12, wobei die Vielzahl von Markierungen jeweils einem Typ des chirurgischen Moduls entspricht, insbesondere einem chirurgischen Modul vom Typ eines regulären oder eines speziellen Forceps oder einem chirurgischen Modul vom Typ einer regulären oder einer speziellen Schere.

14. Augenchirurgisches Instrument nach Anspruch 12 oder 13, wobei die Vielzahl von Markierungen auf dem feststehenden Abschnitt vorgesehen sind.

15. Augenchirurgisches Instrument nach einem der vorstehenden Ansprüche 12 bis 14, wobei der proximale Endabschnitt in Umfangsrichtung arretierbar ist, zumindest nach der selektiven Ausrichtung auf die Markierung.

## Revendications

1. Instrument de chirurgie ophtalmique (1), comprenant :
- une unité de manipulation (2) ayant un axe longitudinal (L),
- un module chirurgical (3) disposé sur l'unité de manipulation (2) et s'étendant à partir de celle-ci le long de son axe longitudinal (L), et
- une tige (4) entourant au moins partiellement le module chirurgical (3), le module chirurgical (3) et la tige (4) étant mutuellement mobiles l'un par rapport à l'autre le long de l'axe longitudinal (L) de l'unité de manipulation (2),
- un élément de guidage (5) disposé à une extrémité distale (6) de l'unité de manipulation (2), l'élément de guidage (5) étant pourvu d'un alésage central (7) qui est traversé par le module chirurgical (3) et la tige s'étendant à partir de l'unité de manipulation vers l'extérieur le long de son axe longitudinal,
dans lequel l'unité de manipulation (2) comprend
- une unité d'entraînement (8) destinée à entraîner le module chirurgical (3) ou la tige (4) par rapport à l'élément de guidage (5) en va-et-vient à travers l'alésage central (7) de l'élément de guidage (5), et
- une unité d'actionnement (9) associée à l'unité d'entraînement (8) pour actionner l'unité d'entraînement (8) lors d'un actionnement manuel de l'unité d'actionnement (9),
dans lequel l'unité d'actionnement (9) comprend un coulisseau distal (10) entourant au moins partiellement l'élément de guidage (5).

2. Instrument de chirurgie ophtalmique selon la revendication 1, dans lequel l'unité d'actionnement est agencée pour déplacer librement le coulisseau distal le long de l'élément de guidage, le long de l'axe longitudinal, lors d'un actionnement manuel de l'unité d'actionnement.

3. Instrument de chirurgie ophtalmique selon l'une quelconque des revendications précédentes, dans lequel l'unité d'actionnement actionne directement le mécanisme d'entraînement.

4. Instrument de chirurgie ophtalmique selon l'une quelconque des revendications précédentes 1 à 3, comprenant en outre une unité de transfert intermédiaire couplée entre l'unité d'actionnement et l'unité d'entraînement.

5. Instrument de chirurgie ophtalmique selon l'une quelconque des revendications précédentes, dans lequel l'élément de guidage présente un contour extérieur radial et dans lequel le coulisseau distal est agencé pour coulisser le long dudit contour extérieur radial le long de l'axe longitudinal lors d'un actionnement manuel de l'unité d'actionnement.

6. Instrument de chirurgie ophtalmique selon l'une quelconque des revendications précédentes, dans lequel l'élément de guidage comprend une cavité alignée avec l'alésage central, et dans lequel l'unité d'entraînement comprend en outre une liaison radiale-vers-axiale comprenant un ensemble d'éléments d'articulation pivotants, chaque élément d'articulation pivotant ayant une première extrémité actionnée par l'unité d'actionnement et une deuxième extrémité associée au module chirurgical ou à la tige, l'unité d'entraînement comprenant en outre un élément de maintien annulaire reçu dans la cavité de l'élément de guidage, l'élément de maintien annulaire étant fixé à demeure au module chirurgical ou à la tige et associé aux deuxièmes extrémités des éléments d'articulation pivotants.

7. Instrument de chirurgie ophtalmique selon l'une quelconque des revendications précédentes, dans lequel le module chirurgical comporte un jonc qui est pourvu, à son extrémité distale, d'une unité opératoire, telle qu'une pince.

8. Instrument de chirurgie ophtalmique selon la revendication 7, dans lequel l'unité d'actionnement comprend une pluralité de bras d'actionnement disposés autour de l'axe longitudinal de l'unité de manipulation, et dans lequel au moins un des bras d'actionnement de l'unité d'actionnement présente un contour extérieur différent d'un contour extérieur d'autres bras d'actionnement, ledit au moins un bras d'actionnement étant aligné avec une orientation circonférentielle de l'unité opératoire du module chirurgical.

9. Module additionnel destiné à être ajouté à un dispositif de chirurgie ophtalmique (1), le dispositif (1) comprenant :
- une unité de manipulation (2) ayant un axe longitudinal (L),
- un module chirurgical (3) disposé sur l'unité de manipulation (2) et s'étendant à partir de celle-ci le long de son axe longitudinal (L), et
- une tige (4) entourant au moins partiellement le module chirurgical (3), le module chirurgical (3) et la tige (4) étant mutuellement mobiles l'un par rapport à l'autre le long de l'axe longitudinal (L) de l'unité de manipulation (2),
- un élément de guidage (5) disposé à une extrémité distale (6) de l'unité de manipulation (2), l'élément de guidage (5) étant pourvu d'un alésage central (7) qui est traversé par le module chirurgical (3) et la tige (4) s'étendant à partir de l'unité de manipulation vers l'extérieur le long de son axe longitudinal,
dans lequel l'unité de manipulation (2) comprend
- une unité d'entraînement (8) destinée à entraîner le module chirurgical (3) ou la tige (4) par rapport à l'élément de guidage (5) en va-et-vient à travers l'alésage central (7) de l'élément de guidage (5), et
- une unité de transfert intermédiaire (20) associée à l'unité d'entraînement (8) pour actionner l'unité d'entraînement (8) lors d'un actionnement de l'unité de transfert intermédiaire (20),
dans lequel le module additionnel comprend une unité d'actionnement (9) à associer à l'unité de transfert intermédiaire (20) pour actionner l'unité de transfert intermédiaire (20) lors d'un actionnement manuel de l'unité d'actionnement (9), et dans lequel l'unité d'actionnement (9) comprend un coulisseau distal (10) entourant au moins partiellement l'élément de guidage (5).

10. Procédé d'assemblage d'un instrument de chirurgie ophtalmique (1), comprenant les étapes consistant à :
- fournir un dispositif de chirurgie ophtalmique, comprenant :
- une unité de manipulation (2) ayant un axe longitudinal (L),
- un module chirurgical (3) disposé sur l'unité de manipulation (2) et s'étendant à partir de celle-ci le long de son axe longitudinal (L), et
- une tige (4) entourant au moins partiellement le module chirurgical (3), le module chirurgical (3) et la tige (4) étant mutuellement mobiles l'un par rapport à l'autre le long de l'axe longitudinal (L) de l'unité de manipulation (2),
- un élément de guidage (5) disposé à une extrémité distale (6) de l'unité de manipulation (2), l'élément de guidage (5) étant pourvu d'un alésage central (7) qui est traversé par le module chirurgical (3) et la tige (4) s'étendant à partir de l'unité de manipulation vers l'extérieur le long de son axe longitudinal,
dans lequel l'unité de manipulation (2) comprend
- une unité d'entraînement (8) destinée à entraîner le module chirurgical (3) ou la tige (4) par rapport à l'élément de guidage (5) en va-et-vient à travers l'alésage central (7) de l'élément de guidage (5), et
- une unité de transfert intermédiaire (20) associée à l'unité d'entraînement (8) pour actionner l'unité d'entraînement (8) lors d'un actionnement de l'unité de transfert intermédiaire (20),
- fournir un module additionnel comprenant une unité d'actionnement (9) à associer à l'unité de transfert intermédiaire (20), l'unité d'actionnement (9) comprenant un coulisseau distal (10) entourant au moins partiellement l'élément de guidage (5), et
- monter le module additionnel sur l'unité de transfert intermédiaire (20) de sorte que, lors d'un actionnement manuel de l'unité d'actionnement (9), l'unité de transfert intermédiaire (20) est actionnée.

11. Procédé de désassemblage d'un instrument de chirurgie ophtalmique, comprenant une étape consistant à retirer le module additionnel défini dans la revendication 9 de l'unité de transfert intermédiaire du dispositif de chirurgie ophtalmique défini dans la revendication 10.

12. Instrument de chirurgie ophtalmique (1), comprenant :
- une unité de manipulation (2) ayant un axe longitudinal (L),
- un module chirurgical (3) disposé sur l'unité de manipulation (2) et s'étendant à partir de celle-ci le long de son axe longitudinal (L), et
- une tige (4) entourant au moins partiellement le module chirurgical (3), le module chirurgical (3) et la tige (4) étant mutuellement mobiles l'un par rapport à l'autre le long de l'axe longitudinal (L) de l'unité de manipulation (2),
- un élément de guidage (5) disposé à une extrémité distale (6) de l'unité de manipulation (2), l'élément de guidage (5) étant pourvu d'un alésage central (7) qui est traversé par le module chirurgical (3) et la tige (4) s'étendant à partir de l'unité de manipulation vers l'extérieur le long de son axe longitudinal,
dans lequel l'unité de manipulation (2) comprend
- une unité d'entraînement (8) destinée à entraîner le module chirurgical (3) ou la tige (4) par rapport à l'élément de guidage (5) en va-et-vient à travers l'alésage central (7) de l'élément de guidage (5),
- une partie stationnaire (31) s'étendant principalement autour de l'axe longitudinal (L), et
- une partie d'extrémité proximale (32) montée rotative sur la partie stationnaire (31), en particulier autour de l'axe longitudinal (L), dans lequel l'une de la partie stationnaire (31) et de la partie d'extrémité proximale (32) est pourvue, le long d'une direction circonférentielle autour de l'axe longitudinal (L), d'un nombre multiple de repères (33), tandis que l'autre de la partie stationnaire (31) et de la partie d'extrémité proximale (32) est pourvue d'un signe d'indication (34) destiné à permettre un alignement sélectif, lors de la rotation de la partie d'extrémité proximale (32) par rapport à la partie stationnaire (31), avec un repère (33) du nombre multiple de repères (33).

13. Instrument de chirurgie ophtalmique selon la revendication 12, dans lequel le nombre multiple de repères correspond respectivement à un type de module chirurgical, en particulier un module chirurgical de type pince ordinaire ou spécialisé, ou un module chirurgical de type ciseaux ordinaire ou spécialisé.

14. Instrument de chirurgie ophtalmique selon la revendication 12 ou 13, dans lequel le nombre multiple de repères est disposé sur la partie stationnaire.

15. Instrument de chirurgie ophtalmique selon l'une quelconque des revendications précédentes 12 à 14, dans lequel la partie d'extrémité proximale est verrouillable dans la direction circonférentielle, au moins après l'alignement sélectif avec le repère.
